# EUROPEAN PATENT APPLICATION

(11) **EP 0 686 401 A2**
(43) Date of publication of application: **13.12.1995**
(21) Application number: 95108752.7
(22) Date of filing: 07.06.1995
(51) Int. Cl.: A61L 25/00, A61K 38/45, A61L 15/38

(54) **Living-tissue adhesive and blood coagulant**

(30) Priority: 10.06.1994 JP 128784/94
(71) Applicant: Ajinomoto Co., Inc., Tokyo 104 (JP)
(72) Inventor: Mawatari, Kazunori, c/o Ajinomoto Co., Inc., Chuo-ku, Tokyo (JP); Usui, Naoki, c/o Ajinomoto Co., Inc., Chuo-ku, Tokyo (JP)
(74) Representative: Strehl Schübel-Hopf Groening & Partner

(57) **Abstract**

Transglutaminase is used as a living tissue adhesive or a blood coagulant.

The transglutaminase crosslinks proteins in body or in blood and brings forth a strong adhesive effect.

## Description

### Field of the Invention

The present invention relates to transglutaminase as an active ingredient in a living-tissue adhesive and in a blood coagulant.

Adhesives such as cyanoacrylate ("Aron Alpha A") and factor XIII have been hitherto used for adhesion of living-tissues (skin, blood vessels, organs and the like).

However, the adhesives such as cyanoacrylate and the like are xenobiotics, and remain in vivo over a long period of time after being used. The adhesives which are used in large quantities sometimes cause the hardening of the adhesion site and the hindrance of adhesion of tissues owing to the heat of polymerization. Moreover, factor XIII alone does not have the ability to cause adhesion of tissues, but promotes adhesion or coating of tissues via the formation of fibrin in combination with fibrinogen, thrombin, aprotinin, calcium chloride and the like. In this case, however, it is indicated that although a mass of fibrin is rapidly formed when using factor XIII, there is a possibility that the tissue and the fibrin mass are easily peeled off.

Thus, the development of a better living-tissue adhesive and blood coagulant which is free from these defects has been in demand.

### Problems To be Solved by the Invention

Under these circumstances, the present invention aims to develop and provide an excellent living-tissue adhesive and an excellent blood coagulant which are free from the above-mentioned defects associated with the conventional living-tissue adhesives.

### Means for Solving the Problems

The present inventor has assiduously conducted investigations to achieve the above-mentioned aim, and has consequently found that a transglutaminase crosslinks proteins in the body or in blood and brings forth a strong adhesive effect. The present invention has been completed on the basis of this finding.

That is, the present invention relates to a transglutaminase for use as an active ingredient in a living-tissue adhesive and in a blood coagulant.

As stated above, the transglutaminase crosslinks body proteins and blood proteins, and brings forth a strong effect of adhesion.

Accordingly, in the adhesion of the living-tissues such as the skin, blood vessels, organs and the like, a protein in the adhesion site is crosslinked with the transglutaminase alone, and the site which is once crosslinked firmly adheres without peeling off. Hence, the use of xenobiotics such as cyanoacrylate and the like, which remain in vivo, is avoided.

Further, in surgical operations involving the blood vessels of the heart or of the liver, there is a need for hemostasis of the bleeding site. In this case, the hemostasis is achieved by forming a fibrin mass upon adding factor XIII to fibrinogen, thrombin, aprotinin, calcium chloride and the like as described above. If one of these ingredients is lacking, the hemostasis does not occur. Meanwhile, transglutaminase has factor XIII activity and also crosslinks with a variety of proteins in plasma. Accordingly, the transglutaminase alone causes the hemostasis. Besides, when the transglutaminase is used as an alternative to factor XIII, not only does it expedite the formation of fibrin, but it crosslinks directly the fibrin mass formed or the blood clot with blood vessels or other living-tissues. For this reason, there is less peeling off at the hemostatic site when transglutaminase is used than another agent that allows only the blood coagulation.

For example, a preparation of a physiological tissue adhesive containing factor XIII of human fibrinogen blood coagulation, aprotinin, thrombin and calcium chloride as active ingredients and further suitable additives has been already commercially available. A preparation which is obtained by replacing factor XIII by transglutaminase either partially or wholly is, of course, within the scope of the present invention.

The transglutaminase which can be employed in the present invention, i.e. the active ingredient of the living-tissue adhesive and the blood coagulant of the present invention, includes a calcium-non-dependent transglutaminase and a calcium-dependent transglutaminase. The former includes a transglutaminase of a microorganism (see, for example, Japanese Laid-Open Patent Application (Kokai) No. 64-27,471). The latter includes a transglutaminase of a guinea pig (see, for example, Japanese Patent Publication (Kokoku) No. 1-50,382) and a transglutaminase of fish (see, for example, Seki Nobuo, et al., "Preprint of 1988 Fall Meeting of Japan Fishery Academy", p. 167 and "Preprint of 1990 Spring Meeting of Japan Fishery Academy", p. 219). Of these, the calcium-non-dependent transglutaminase is preferable, and it can be produced by a method described in, for example, the above-mentioned Japanese Laid-Open Patent Application (Kokai) No. 64-27,471.

The living-tissue adhesive and the blood coagulant of the present invention may contain, in addition to the transglutaminase which is the essential active ingredient, additives which are ordinarily used in these agents, such as a stabilizer, a solubilizing agent, a pH buffer and the like, as required.

The living-tissue adhesive and the blood coagulant of the present invention can consist of the transglutaminase as such, or can be a solution containing the TG, or can be a preparation for local administration containing the transglutaminase and using an excipient or adjuvant in a solution, a semi-solution, a gel or dispersion. A solution, an ointment, a local protective agent such as a gauze and a spray are preferable from the standpoint of administration. The solution can be prepared by dissolving the transglutaminase in a solvent such as sterile water, a buffer solution or the like. The ointment is not particularly limited with respect to the substrate to be used. The substrate can be formed appropriately from an ordinary starting material such as a fat, fatty oil, lanolin, vaseline, paraffin, wax, plaster, resin, plastics, glycol, higher alcohol, glycerin, water, emulsifying agent or suspending agent. The content of the transglutaminase in the solution, ointment, local protective agent or spray is not particularly limited either. It is, however, advantageous in the administration that the content of the transglutaminase is from 0.1 to 1,000 U/g, preferably from 0.5 to 500 U/g.

Needless-to-say, it is advisable that the solution containing the agent of the present invention be once freeze-dried when the agent is put to a distribution system. In practical use, the freeze-dried product is returned to an aqueous solution with, for example, water. The above-mentioned content of the transglutaminase in the freeze-dried product is the content in the aqueous solution obtained by returning the freeze-dried product with water.

As a result of the test, the living-tissue adhesive and the blood coagulant of the present invention did not exhibit acute toxicity.

The living-tissue adhesive and the blood coagulant of the present invention are used not only in humans but also in other animals.

### Examples

The present invention will be illustrated more specifically by referring to the following Examples.

### Example 1 (Effect of adhesion of a living-tissue)

Ten six-week-old SD-strain SPF male rats (supplied by Japan Charles River K.K.) were obtained. These rats were kept in quarantine and bred for approximately 1 week. When the rats were seven weeks old, they were subjected to the experiment.

The rats were anesthetized with pet barbital Na salt (40 mg/kg, i.p.). Then, the hair on a section of the skin from the backs of the rats was removed by means of a shaver (approximately 3 cm x 7 cm). The portion from which the hair was removed was sterilized with hibitane and cut by means of a surgical knife to provide a cut having a depth of approximately 5 mm and a length of approximately 5 cm.

After the cut sample underwent hemostasis, a physiological saline solution was applied on the cut sample of the first group (consisting of 5 rats), and was fixed with a plaster. Further, an aqueous solution (20 U/ml) of a transglutaminase of a microorganism produced by the method in Example 1 of Japanese Laid-Open Patent Application No. 64-27,401 was applied on the cut sample of the second group (consisting of the 5 rats), and was fixed similarly with a plaster. Then, 3 hours later, the plaster was peeled off, and the cut was manually pulled right and left to confirm whether the cut had adhered or not.

The results to confirm the adhesion are shown in Table 1.

**Table 1**

| First group (group on which a physiological saline solution was applied): | |
|---|---|
| Test animal No. | Results |
| 1 | The cut was opened right and left with a light force and was bled. |
| 2 | - ditto - |
| 3 | - ditto - |
| 4 | - ditto - |
| 5 | - ditto - |

| Second group (group on which a transglutaminase was applied): | |
|---|---|
| Test animal No. | Results |
| 1 | The cut was completely adhered without being open. |
| 2 | The cut was adhered but partially opened. |
| 3 | The cut was completely adhered without being open. |
| 4 | The cut was completely adhered without being open. |
| 5 | The cut was completely adhered without being open. |

As shown in Table 1, in the physiological saline solution-applied group, the cut of any of the test animals was opened right and left with the light force and bled. Meanwhile, in the transglutaminase-applied group, the cut was adhered 3 hours after the coating. When force was exerted right and left on the cut, the cut of one test animal was slightly opened, but the cuts of all the other test animals were almost completely adhered without opening.

### Example 2 (Effect of blood coagulation)

### (a) Measurement of blood coagulation time (Lee-White method):

According to a Lee-White method, a blood coagulation time was measured as follows. That is:
(1) Two small test tubes 10 mm in inner diameter and 100 mm in length were warmed in a constant temperature tank of 37°C.
(2) Using a dry-sterilized syringe equipped with a 21G disposable injection needle, a blood sample was collected from the aorta celiaca of a rat. The moment the blood flowed into the syringe, a microchronometer began to move.
(3) One milliliter of the sample blood was added to each of the two warmed small test tubes.
(4) After the test tubes stood still for 3 minutes, 20 µl of a "Fibrin Glue" solution A, 20 µl of a "Fibrin Glue" solution B and 1.2 U of a transglutaminase solution or same volume of a physiological saline solution instead of transglutaminase solution were added to each of the test tubes.
(5) Thereafter, rapidly, the one test tube was put in an inclined position every 30 seconds to observe the flowability of the blood.
(6) When the flowability disappeared and the blood was coagulated, the second test tube was subjected to the same procedure.
(7) When the flowability disappeared in the second test tube, the microchronometer was stopped; the time was read and it was documented as the coagulation time. This measurement was repeated for a total of three times.

The "Fibrin Glue" (made by Hoechst Japan) solution A was obtained by dissolving a fibrinogen dry powder containing factor XIII with an aprotinin solution heated to from 35 to 37°C. The "Fibrin Glue" solution B was obtained by dissolving a thrombin powder with a calcium chloride solution. The "Fibrin Glue" solutions A and B were applied on the bleeding portion either singly or simultaneously.

The results are shown in Table 2.

**Table 2**

| A solution | B solution | TG | 1st | 2nd | 3rd | Average |
|---|---|---|---|---|---|---|
| 20 µl | 20 µl | 0 U | 4 min | 4 min | 4 min | 4 min |
| 20 µl | 20 µl | 1.2 U | 3.5 min | 3.5 min | 3.5 min | 3.5 min |

Three minutes after the collection of the sample blood, the transglutaminase solution or the physiological saline solution was added in addition the above-mentioned "Fibrin Glue" A and B solutions. Then, it was found that the blood coagulation time was shortened in the area to which the transglutaminase solution was added.

### (b) Measurement of blood coagulability:

Blood coagulability was measured as follows. That is:
(1) A 10-cm² parafilm was attached to a test stand.
(2) Twenty microliters of the transglutaminase solution (20 U/20 µl) or the physiological saline solution, 20 µl of the "Fibrin Glue" solution A, 20 µl of the "Fibrin Glue" solution B and 20 µl of a blood sample collected from the arteria subclavia of a rat were dropped on the parafilm longitudinally at intervals of 8 mm.
(3) The piston portion of a 2-milliliter syringe was drastically reciprocated longitudinally along the dropped solution group, and the solution was stirred for 20 seconds.
(4) The amount and strength of the gel formed were observed.

The results were as follows. That is, in the area to which the transglutaminase was added, the amount of the gel was approximately 50 µl. Whereas, in the area to which the transglutaminase was not added, the amount of the gel was less than approximately 20 µl. Further, in the area to which the transglutaminase was added, the gel could be extended. Whereas, in the area to which the transglutaminase was not added, the gel could not be extended.

### Effects of the Invention

The present invention can easily provide a novel, excellent living-tissue adhesive comprising a transglutaminase as an active ingredient and a novel, excellent blood coagulant comprising a transglutaminase as an active ingredient.

## Claims

1. A transglutaminase for use as a living tissue adhesive.

2. A transglutaminase for use as a blood coagulant.

3. A transglutaminase of claim 1 or claim 2, wherein said transglutaminase is calcium-non-dependent.
